# FASCICULE DE BREVET EUROPEEN

(11) **EP 1 984 048 B1**
(45) Date de publication et mention de la délivrance du brevet: **18.04.2018**
(21) Numéro de dépôt: 07731508.3
(22) Date de dépôt: 23.01.2007
(51) Int. Cl.: A61M 15/00

(54) **EMBOUT BUCCAL POUR DISPOSITIF DE DISTRIBUTION DE PRODUIT FLUIDE**
MUNDSTÜCK FÜR EINE VORRICHTUNG ZUM ABGEBEN EINES FLÜSSIGEN PRODUKTS
MOUTHPIECE FOR A DEVICE FOR DISPENSING A FLUID PRODUCT

(30) Priorité: 25.01.2006 FR 0650265
(43) Date de publication de la demande: 29.10.2008
(73) Titulaire: Aptar France SAS, 27110 Le Neubourg (FR)
(72) Inventeur: POCOCK, Andrew Gordon, Cambridge Cambridgeshire CB10 1SX (GB); KAY, Stuart Brian William, Cambridge Cambridgeshire CB10 1SX (GB); GREENHALGH, Paul, Cambridge Cambridgeshire CB10 1SX (GB); O'HARA, Wayne, Cambridge Cambridgeshire CB10 1SX (GB)
(74) Mandataire: CAPRI
(86) Numéro de dépôt international: PCT/FR2007/050679
(87) Numéro de publication internationale: WO 2007/085758

(56) Documents cités:
- WO-A-2005/014089
- WO-A-2006/079749
- US-A- 1 507 571
- US-A- 4 263 907
- US-A- 5 829 434
- US-A1- 2003 178 024

## Description

La présente invention concerne un embout buccal pour dispositif de distribution de produit fluide, ainsi qu'un tel dispositif de distribution de produit fluide, en particulier un inhalateur de poudre sèche.

Les inhalateurs de poudre sèche sont bien connus dans l'état de la technique. Il en existe différentes sortes. Un point commun à tous les inhalateurs est qu'ils comportent un orifice de distribution à travers lequel l'utilisateur reçoit la poudre distribuée. Généralement, cet orifice de distribution est prévu sur un embout buccal sur ou autour duquel l'utilisateur place sa bouche pour actionner l'inhalateur et recevoir la dose de poudre. Selon la structure de l'inhalateur, divers inconvénients peuvent apparaître au niveau de l'embout buccal. D'une part, certains embouts buccaux, notamment de petite dimension, présentent un risque en ce que l'utilisateur peut s'il ne fait pas attention placer ses dents au moins partiellement devant l'ouverture ou l'orifice de distribution, empêchant ainsi une distribution de la totalité de la dose lors de l'actionnement de l'inhalateur. Par ailleurs, certains embouts buccaux ne sont pas toujours ergonomiques et ne garantissent pas une bonne étanchéité avec la bouche, présentant donc un risque à la fois de fuite du produit expulsé par l'inhalateur mais également un risque de perte de charge au moment de l'inhalation, la totalité de l'effort d'inhalation de l'utilisateur n'étant pas dirigé à l'intérieur de l'appareil. De plus, pour les inhalateurs comportant des éléments de capot adaptés à couvrir et découvrir l'orifice de distribution selon que le capot est en position d'ouverture ou de fermeture, il existe généralement un risque pour l'utilisateur de se coincer un ou plusieurs doigt(s) au niveau de l'embout buccal lors principalement de la fermeture desdits éléments de capot. Les documents US 2003/178024, US-5 829 434 et WO 2005/014089 divulguent des inhalateurs de l'état de la technique.

La présente invention a pour but de fournir un embout buccal pour dispositif de distribution de produit fluide qui ne reproduit pas les inconvénients susmentionnés.

En particulier, la présente invention a pour but de fournir un embout buccal qui soit ergonomique, qui permette une bonne étanchéité avec la bouche de l'utilisateur, qui évite sensiblement tout risque d'obstruction de l'orifice de distribution par les dents de l'utilisateur lors de l'inhalation, et qui élimine sensiblement tout risque de coincement des doigts lors de la manipulation du ou des capot(s) mobile(s) de l'inhalateur.

La présente invention a également pour but de fournir un embout buccal qui soit peu coûteux à fabriquer et à assembler et simple d'utilisation.

La présente invention a également pour but de fournir un dispositif de distribution de produit fluide comportant un tel embout buccal.

La présente invention a donc pour objet un embout buccal pour dispositif de distribution de produit fluide, comportant un corps pourvu d'un orifice de distribution de produit fluide à travers lequel le produit fluide est expulsé, ledit corps présentant une forme allongée sensiblement bombée avec une surface supérieure incorporant ledit orifice de distribution et s'étendant longitudinalement des deux côtés dudit orifice de distribution, deux surfaces d'appui latérales étant prévues latéralement des deux côtés dudit orifice de distribution pour recevoir les lèvres de l'utilisateur lors de l'inhalation, ladite surface supérieure étant légèrement convexe et lesdites surfaces d'appui latérales étant légèrement concaves.

Avantageusement, lesdites surfaces d'appui latérales prolongent latéralement ladite surface supérieure.

Avantageusement, la forme des surfaces d'appui latérales oblige l'utilisateur à ouvrir suffisamment la bouche pour que ses dents ne bloquent pas l'orifice de distribution lors de la distribution du produit fluide.

Avantageusement, la forme générale du corps est convexe.

Avantageusement, lesdites surfaces d'appui latérales s'étendent longitudinalement sur une partie centrale dudit corps de manière symétrique par rapport à l'orifice de distribution.

Avantageusement, lesdites surfaces d'appui latérales s'étendent longitudinalement sur la plus grande partie dudit corps.

Avantageusement, lesdites surfaces d'appui latérales sont séparées par ladite surface supérieure et ledit orifice de distribution.

Avantageusement, ledit orifice de distribution définit un canal central d'expulsion de produit fluide et un canal d'air co-axial disposé autour dudit canal central pour créer l'écoulement d'air lors de l'inhalation.

La présente invention a également pour objet un dispositif de distribution de produit fluide comprenant un embout buccal tel que décrit ci-dessus.

Avantageusement, ledit embout buccal est encliqueté sur un corps dudit dispositif.

Avantageusement, le dispositif comporte au moins un élément de capot mobile entre une position de fermeture dans laquelle il recouvre l'orifice de distribution et une position d'ouverture dans laquelle il découvre l'orifice de distribution, la forme dudit embout buccal empêchant tout coincement des doigts de l'utilisateur lors de l'ouverture et/ou la fermeture dudit au moins un élément de capot.

Avantageusement, ledit dispositif est un inhalateur de poudre sèche.

Ces caractéristiques et avantages et d'autres de la présente invention apparaîtront plus clairement au cours de la description détaillée suivante d'un mode de réalisation particulier de celle-ci, fait en référence aux dessins joints, donnés à titre d'exemple non limitatif, et sur lesquels
- la figure 1 est une vue schématique en perspective d'un inhalateur de poudre sèche selon un mode de réalisation particulier de la présente invention ;
- la figure 2 est une vue schématique partielle en section transversale d'un système de déclenchement par inhalation, selon un mode de réalisation particulier, en position avant inhalation,
- la figure 3 est une vue similaire à celle de la figure 2, après inhalation,
- la figure 4 est une vue schématique en perspective d'un embout buccal selon un mode de réalisation avantageux de la présente invention,
- la figure 5 est une vue de dessus agrandie de l'embout buccal de la figure 4, et
- la figure 6 est une vue en section transversale de côté de l'embout buccal des figures 4 et 5.

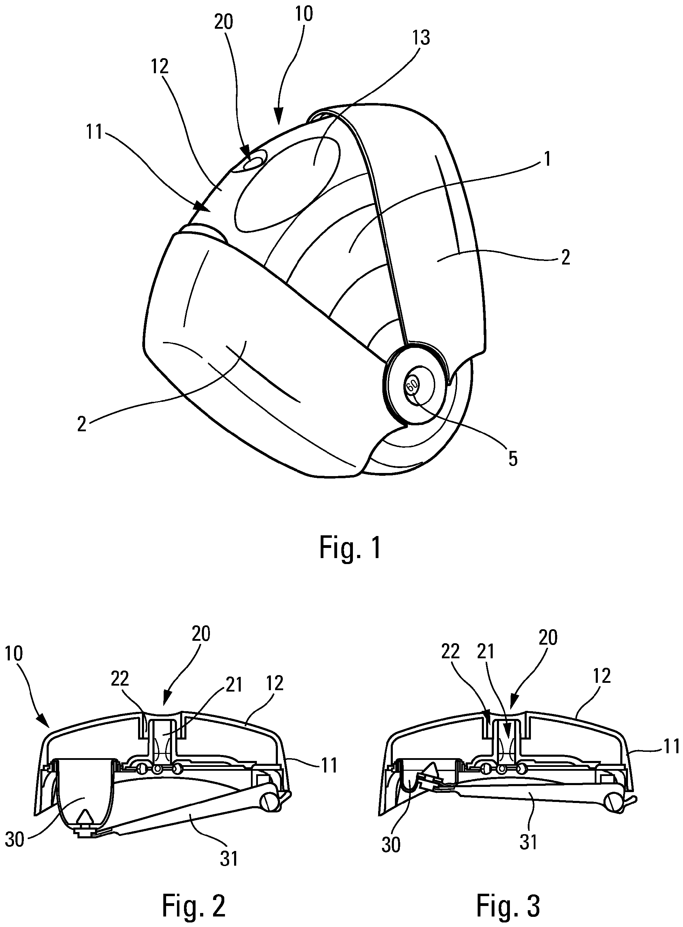

La figure 1 représente un exemple d'inhalateur de poudre sèche auquel la présente invention s'applique. Il est toutefois entendu que l'inhalateur représenté sur cette figure 1 n'est pas limitatif, et qu'au contraire l'embout buccal de l'invention pourrait s'appliquer à tout type d'inhalateur de poudre sèche de forme quelconque. De manière générale, l'embout buccal de la présente invention s'applique à la distribution de tous types de produit fluide, c'est-à-dire liquide, pâteux, pulvérulent ou gazeux.

L'inhalateur représenté sur la figure 1 comporte un corps 1 sur lequel sont assemblés deux éléments de capot mobile 2, qui dans cet exemple pivotent autour d'un axe de pivotement commun entre une position d'ouverture (représentée sur la figure 1), dans laquelle un orifice de distribution 20 de l'inhalateur est découvert, et une position de fermeture (non représentée), dans laquelle les deux éléments de capot mobile 2 sont ramenés l'un contre l'autre, pour recouvrir l'orifice de distribution. Avantageusement, un compteur de doses peut être prévu dans l'inhalateur, et les doses peuvent s'afficher dans une fenêtre 5 qui peut par exemple être prévue au niveau de l'axe de pivotement des deux éléments de capot 2, comme représenté schématiquement sur la figure 1.

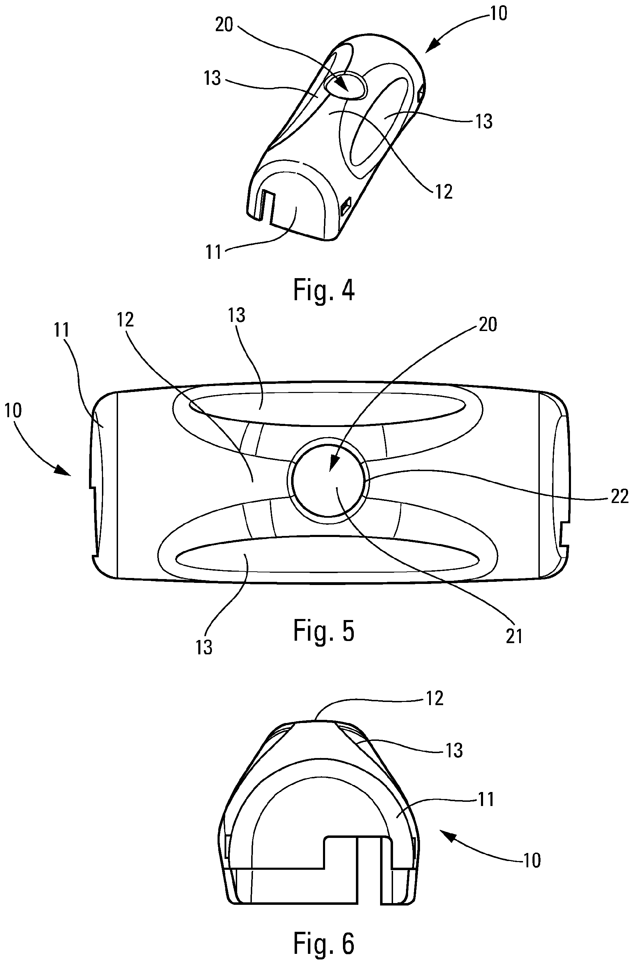

Les figures 4 à 6 représentent un mode de réalisation avantageux de l'embout buccal de la présente invention. En se référant à ces figures 4 à 6, l'embout buccal 10 comporte un corps 11 qui est pourvu d'un orifice de distribution de produit fluide 20. Dans le cas d'un inhalateur, le produit fluide est généralement de la poudre sèche mélangée à un écoulement d'air. Dans le cadre d'un inhalateur dit passif, l'écoulement d'air est celui crée par l'inhalation de l'utilisateur. Dans le cas d'un inhalateur dit actif, un écoulement d'air comprimé est utilisé pour expulser la poudre au moment de l'actionnement de l'inhalateur. Comme visible notamment sur la figure 4, le corps 11 présente une forme longitudinalement allongée sensiblement bombée ou convexe dans la direction transversale avec une surface supérieure 12 qui incorpore l'orifice de distribution 20, de préférence en son centre. De manière générale, l'embout buccal 10 est de préférence symétrique par rapport audit orifice de distribution 20. La surface supérieure 12 s'étend longitudinalement des deux côtés dudit orifice de distribution 20 et avantageusement jusqu'aux bords latéraux du corps 11. On constate que cette surface supérieure 12 est également légèrement bombée ou convexe dans la direction longitudinale, octroyant ainsi à l'embout buccal 10 une forme arrondie aplatie. De part et d'autre de l'orifice de distribution 20, dans la direction transversale ou latérale, le corps 11 comporte deux surfaces d'appui latérales 13 respectivement prévues latéralement de chaque côté dudit orifice de distribution 20. Chacune de ces surfaces d'appui latérales 13 est destinée à recevoir une lèvre de l'utilisateur lors de l'inhalation. Ces surfaces d'appui latérales 13 sont légèrement concaves, notamment dans la direction transversale, pour former de légers creux adaptés à bien positionner les lèvres de l'utilisateur lorsque celui-ci les place sur l'embout buccal. Comme visible notamment sur les figures, les surface d'appui latérales 13 prolongent latéralement ladite surface supérieure 12, et sont de préférence séparées l'une de l'autre par cette surface supérieure 12, ainsi que par l'orifice de distribution 20. De cette manière, lorsque l'utilisateur place sa bouche sur l'embout buccal 10, l'écartement entre les deux surfaces d'appui latérales 13 oblige l'utilisateur à ouvrir suffisamment la bouche pour éviter que ses dents ne viennent obturer même partiellement l'orifice de distribution 20 lors de l'inhalation. Par ailleurs, la surface plane ou légèrement concave des surfaces d'appui latérales 13 garantit une bonne ergonomie et assure une bonne étanchéité de la bouche de l'utilisateur sur l'embout buccal 10 au moment de l'inhalation. Dans le cas d'un inhalateur de type passif, ceci évite une perte de charge inutile lors de l'inhalation et garantit que la plus grande partie possible de l'écoulement d'inhalation crée par l'utilisateur sera utilisée pour expulser la poudre contenue dans l'inhalateur. L'écartement et la profondeur des surfaces d'appui latérales 13 garantit également que l'orifice de distribution se positionne à l'intérieur de la bouche, au-delà des dents, sans pour autant que cet orifice de distribution 20 soit formé sur une partie saillante de l'embout buccal 10.

Avantageusement, les surfaces d'appui latérales 13 s'étendent longitudinalement sur une partie centrale dudit corps 11, de préférence sur la plus grande partie de celui-ci, comme cela est notamment visible sur la figure 5. Avantageusement, ces deux surfaces d'appui latérales 13 sont parfaitement symétriques l'une par rapport à l'autre par rapport à l'orifice de distribution 20.

Avantageusement, l'orifice de distribution 20 définit un canal central d'expulsion de poudre 21 et un canal d'air 22, co-axial par rapport audit canal central 21, et disposé autour de celui-ci. Le canal d'air 22 permet de créer l'écoulement d'air lors de l'inhalation et, dans le cas d'un inhalateur déclenché par l'inhalation de l'utilisateur, il permet d'actionner le système de déclenchement, comme cela sera décrit ci-après en référence aux figures 2 et 3.

En se référant à nouveau à la figure 1, on constate que les éléments de capot 2 mobiles autour du corps 1 de l'inhalateur et de l'embout buccal 10, comportent une surface supérieure de forme bombée sensiblement adaptée à celle de l'embout buccal, notamment à sa surface supérieure 12. De cette manière, lors du déplacement desdits éléments de capot 2 entre la position d'ouverture et de fermeture, aucun espace n'est crée par la présence de l'embout buccal 10, de sorte que l'utilisateur ne risque pas de se coincer les doigts lors de la manipulation de ces éléments de capot 2. De manière générale, l'embout buccal de la présente invention ne comporte aucune partie saillante qui risquerait de créer un interstice pouvant recevoir des doigts de l'utilisateur lors de l'actionnement du dispositif.

En se référant maintenant aux figures 2 et 3, il y est représenté un système de déclenchement par inhalation d'un inhalateur selon un mode de réalisation avantageux. Ce système comporte avantageusement une chambre déformable 30, également appelée diaphragme qui, lorsque l'utilisateur inhale à travers l'embout buccal 20 va se déformer, pour déplacer une tige 31 reliée audit diaphragme 30 et ainsi déclencher l'expulsion d'une dose de poudre. En se référant aux figures 2 et 3, on constate que le canal d'air 22 disposé dans l'orifice de distribution 20 autour du canal central d'expulsion de poudre 21 est directement relié à ladite chambre déformable 30 ce qui permet d'assurer un déclenchement fiable du dispositif. Les figures 2 et 3 représentent également la forme bombée ou convexe en direction longitudinale de la surface supérieure 12 de l'embout buccal ainsi que le fait que l'orifice de distribution 20 de cet embout buccal n'est pas formé sur une partie saillante par rapport à ladite surface supérieure 12 du corps 11.

Bien que l'invention ait été décrite en référence à un mode de réalisation particulier de celle-ci, il est entendu qu'elle n'est pas limitée par les exemples représentés. Au contraire, un homme du métier peut y apporter toutes modifications utiles sans sortir du cadre de la présente invention tel que défini par les revendications annexées.

## Revendications

1. Embout buccal (10) pour dispositif de distribution de produit fluide, comportant un corps (11) pourvu d'un orifice de distribution de produit fluide (20) à travers lequel le produit fluide est expulsé, ledit corps (11) présentant une forme allongée sensiblement bombée avec une surface supérieure (12) incorporant ledit orifice de distribution (20) et s'étendant longitudinalement des deux côtés dudit orifice de distribution (20), deux surfaces d'appui latérales (13) étant prévues latéralement des deux côtés dudit orifice de distribution (10) pour recevoir les lèvres de l'utilisateur lors de l'inhalation, ladite surface supérieure (12) étant légèrement convexe **caractérisé en ce que** lesdites surfaces d'appui latérales (13) sont légèrement concaves.

2. Embout buccal selon la revendication 1, dans lequel lesdites surfaces d'appui latérales (13) prolongent latéralement ladite surface supérieure (12).

3. Embout buccal selon l'une quelconque des revendications précédentes, dans lequel la forme générale du corps (11) est convexe.

4. Embout buccal selon l'une quelconque des revendications précédentes, dans lequel lesdites surfaces d'appui latérales (13) s'étendent longitudinalement sur une partie centrale dudit corps (11) de manière symétrique par rapport à l'orifice de distribution (20).

5. Embout buccal selon la revendication 4, dans lequel lesdites surfaces d'appui latérales (13) s'étendent longitudinalement sur la plus grande partie dudit corps (11).

6. Embout buccal selon l'une quelconque des revendications précédentes, dans lequel lesdites surfaces d'appui latérales (13) sont séparées par ladite surface supérieure (12) et ledit orifice de distribution (20), de sorte que l'utilisateur est obligé d'ouvrir suffisamment la bouche pour que ses dents ne bloquent pas l'orifice de distribution (20) lors de la distribution du produit fluide.

7. Embout buccal selon l'une quelconque des revendications précédentes, dans lequel ledit orifice de distribution (20) définit un canal central d'expulsion de produit fluide (21) et un canal d'air co-axial (22) disposé autour dudit canal central (21) pour créer l'écoulement d'air lors de l'inhalation.

8. Dispositif de distribution de produit fluide, **caractérisé en ce qu'**il comprend un embout buccal (10) selon l'une quelconque des revendications précédentes.

9. Dispositif selon la revendication 8, dans lequel ledit embout buccal (10) est encliqueté sur un corps (1) dudit dispositif.

10. Dispositif selon la revendication 8 ou 9, dans lequel le dispositif comporte au moins un élément de capot (2) mobile entre une position de fermeture dans laquelle il recouvre l'orifice de distribution (20) et une position d'ouverture dans laquelle il découvre l'orifice de distribution (20), la forme dudit embout buccal (10) empêchant tout coincement des doigts de l'utilisateur lors de l'ouverture et/ou la fermeture dudit au moins un élément de capot (2).

11. Dispositif selon l'une quelconque des revendications 8 à 10, dans lequel ledit dispositif est un inhalateur de poudre sèche.

## Patentansprüche

1. Mundansatz (10) für eine Ausgabevorrichtung für ein fluides Produkt, aufweisend einen Körper (11), der mit einer Ausgabeöffnung für fluides Produkt (20) versehen ist, durch die das fluide Produkt ausgestoßen wird, wobei der Körper (11) eine langgestreckte, im Wesentlichen bauchige Form mit einer oberen Oberfläche (12) aufweist, in der die Ausgabeöffnung (20) ausgebildet ist und die sich längs zu beiden Seiten der Ausgabeöffnung (20) erstreckt, wobei zwei seitliche Stützflächen (13) seitlich der beiden Seiten der Ausgabeöffnung (10) vorgesehen sind, um die Lippen des Benutzers bei der Inhalation aufzunehmen, wobei die obere Oberfläche (12) leicht konvex ist, **dadurch gekennzeichnet, dass** die seitlichen Stützflächen (13) leicht konkav sind.

2. Mundansatz nach Anspruch 1, wobei die seitlichen Stützflächen (13) die obere Oberfläche (12) seitlich verlängern.

3. Mundansatz nach einem der vorhergehenden Ansprüche, wobei die allgemeine Form des Körpers (11) konvex ist.

4. Mundansatz nach einem der vorhergehenden Ansprüche, wobei sich die seitlichen Stützflächen (13) längs auf einem mittleren Teil des Körpers (11) symmetrisch in Bezug auf die Ausgabeöffnung (20) erstrecken.

5. Mundansatz nach Anspruch 4, wobei sich die seitlichen Stützflächen (13) auf dem größeren Teil des Körpers (11) längs erstrecken.

6. Mundansatz nach einem der vorhergehenden Ansprüche, wobei die seitlichen Stützflächen (13) durch die obere Oberfläche (12) und die Ausgabeöffnung (20) derart getrennt sind, dass der Benutzer gezwungen ist, den Mund ausreichend zu öffnen, damit seine Zähne bei der Ausgabe des fluiden Produkts nicht die Ausgabeöffnung (20) blockieren.

7. Mundansatz nach einem der vorhergehenden Ansprüche, wobei die Ausgabeöffnung (20) einen mittleren Austreibkanal für fluides Produkt (21) und einen koaxialen Luftkanal (22) definiert, der um den mittleren Kanal (21) angeordnet ist, um bei der Inhalation den Luftstrom zu erzeugen.

8. Ausgabevorrichtung für ein fluides Produkt, **dadurch gekennzeichnet, dass** sie einen Mundansatz (10) nach einem der vorhergehenden Ansprüche aufweist.

9. Vorrichtung nach Anspruch 8, wobei der Mundansatz (10) auf einem Körper (1) der Vorrichtung eingerastet ist.

10. Vorrichtung nach Anspruch 8 oder 9, wobei die Vorrichtung mindestens ein Kappenelement (2) aufweist, das zwischen einer Schließposition, in der sie die Ausgabeöffnung (20) bedeckt, und einer Öffnungsposition, in der sie die Ausgabeöffnung (20) freigibt, beweglich ist, wobei die Form des Mundansatzes (10) ein Einklemmen der Finger des Benutzers beim Öffnen und/oder Schließen des mindestens einen Kappenelements (2) verhindert.

11. Vorrichtung nach einem der Ansprüche 8 bis 10, wobei die Vorrichtung ein Inhalator für Trockenpulver ist.

## Claims

1. A mouthpiece (10) for a fluid dispenser device, comprising a body (11) that is provided with a fluid dispenser orifice (20) through which the fluid is expelled, said body (11) presenting a substantially rounded elongate shape, with a top surface (12) incorporating said dispenser orifice (20) and extending longitudinally on both sides of said dispenser orifice (20), two lateral bearing surfaces (13) being provided laterally on both sides of said dispenser orifice (10) for receiving the lips of the user during inhalation, said top surface (12) being slightly convex **characterized in that** said lateral bearing surfaces (13) are slightly concave.

2. A mouthpiece according to claim 1, in which said lateral bearing surfaces (13) extend said top surface (12) laterally.

3. A mouthpiece according to any preceding claim, in which the general shape of the body (11) is convex.

4. A mouthpiece according to any preceding claim, in which said lateral bearing surfaces (13) extend longitudinally over a central portion of said body (11), symmetrically about the dispenser orifice (20).

5. A mouthpiece according to claim 4, in which said lateral bearing surfaces (13) extend longitudinally over the majority of said body (11).

6. A mouthpiece according to any preceding claim, in which said lateral bearing surfaces (13) are separated by said top surface (12) and by said dispenser orifice (20), such that the user is obliged to open the mouth wide enough for the teeth not to block the dispenser orifice (20) while the fluid is being dispensed.

7. A mouthpiece according to any preceding claim, in which said dispenser orifice (20) defines a central fluid-expulsion channel (21) and a coaxial air channel (22) that is disposed around said central channel (21) so as to create a flow of air during inhalation.

8. A fluid dispenser device, **characterized in that** it includes a mouthpiece (10) according to any preceding claim.

9. A device according to claim 8, in which said mouthpiece (10) is snap-fastened on a body (1) of said device.

10. A device according to claim 8 or claim 9, in which the device includes at least one cover element (2) that is movable between a closed position in which it covers the dispenser orifice (20) and an open position in which it uncovers the dispenser orifice (20), the shape of said mouthpiece (10) preventing the fingers of the user from being pinched while said at least one cap element (2) is being opened and/or closed.

11. A device according to any one of claims 8 to 10, in which said dispenser is a dry-powder inhaler.
